# EUROPEAN PATENT SPECIFICATION

(11) **EP 1 128 822 B1**
(45) Date of publication and mention of the grant of the patent: **12.10.2005**
(21) Application number: 99956311.7
(22) Date of filing: 05.11.1999
(51) Int. Cl.: A61K 31/22, A61K 31/205, A61K 35/78, A23L 1/302, A23L 1/304, A61P 9/10, A61P 37/08, A61P 29/00

(54) **ANTIOXIDANT COMPOSITIONCOMPRISING PROPIONYL L-CARNITINE AND A FLAVONOID AGAINST THROMBOSIS AND ATHEROSCLEROSIS**
ANTIOXIDATIVE ZUSAMMENSETZUNG ENTHALTEND PROPIONYL L-CARNITIN UND EIN FLAVONOID GEGEN THROMBOSE UND ATHEROSKLEROSE
COMPOSITION ANTIOXYDANTE RENFERMANT DE LA L-CARNITINE DE PROPIONYLE ET UN FLAVONOIDE POUR LUTTER CONTRE LA THROMBOSE ET L'ATHEROSCLEROSE

(30) Priority: 13.11.1998 IT RM980706
(43) Date of publication of application: 05.09.2001
(73) Proprietor: SIGMA-TAU Industrie Farmaceutiche Riunite S.p.A., 00144 Roma (IT)
(72) Inventor: CAVAZZA, Claudio, I-00186 Roma (IT)
(74) Representative: Cavattoni, Fabio
(86) International application number: PCT/IT1999/000351
(87) International publication number: WO 2000/028986

(56) References cited:
- EP-A- 0 928 565
- EP-A- 0 945 126
- WO-A-96/36348
- WO-A-97/04668
- WO-A-98/33494
- WO-A-99/58000
- WO-A-99/66913
- DE-A- 19 806 890
- US-A- 5 976 568
- "Kwik Burn. Thermogenic intensifier" NUTRITION.COM, [Online] XP002133135 Retrieved from the Internet: <URL:http://www.1nutrition.com/products/La brada/kwik_burn.htm> [retrieved on 2000-03-15]
- "Metacuts. Energy thermogenic" HEALTHNESS.COM, [Online] XP002133136 Retrieved from the Internet: <URL:http://www.healthness.com/metacuts.ht m> [retrieved on 2000-03-15]
- KUKREJA R.C. ET AL: "Activated oxygen species in heart failure." HEART FAILURE REVIEWS, (1999) 4/2 (121-132). , XP002133137

## Description

The present invention relates to the use of a composition for the manufacture of a medicament or dietary supplement for the prevention and/or treatment of thrombotic or atherosclerotic abnormalities, allergic inflammatory reactions, diseases brought about by the release of free radicals and by increased platelet aggregation.

Accordingly, the composition may take the form and exert the action of a dietary supplement or of an actual medicine, depending upon the support or preventive action, or the strictly therapeutic action, which the composition is intended to exert in relation to the particular individuals it is to be used in.

More particularly the present invention relates to the use of an orally, parenterally, rectally, cutaneously or transdermally administrable composition which comprises in combination:
(a) propionyl L-carnitine or a pharmacologically acceptable salt thereof, possibly in combination with another "carnitine", where what is meant by "carnitine" is L-carnitine or an alkanoyl L-carnitine selected from the group comprising acetyl L-carnitine, valeryl L-carnitine and isovaleryl L-carnitine or their pharmacologically acceptable salts; and
(b) a flavonoid, preferably selected from the group comprising quercetin, rutin, myricetin, myricitrin or mixtures thereof or extracts of natural vegetable products containing such flavonoids.

Here below, for the sake of brevity and simplicity of presentation, reference will be made only to quercetin as an example of a flavonoid, it being understood that the description applies equally to the other flavonoids mentioned in the present invention.

Quercetin is a naturally occurring product belonging to the group of the polyphenolic flavonoids and is present in many vegetable and plant foods such as apples, garlic, grapes and wine, hazel nuts and tea-leaves.

Quercetin is most often present in its conjugate form with glucose as glucoside or as 3-rutinoside (rutin), forms which are capable of conditioning its intestinal absorption after ingestion into the body with food. Apples contain various quercetin glucosides, including galactosides, xylosides, arabinosides, rhamnosides and glycosides. Tea-leaves contain mainly quercetin rutinosides, whereas the quercetin derivatives contained in garlic are mainly glycosides.

In grapes and wine, on the other hand, quercetin is found both as glycoside and as aglycone.

Quercetin belongs to the group of flavonoids that recent epidemiological studies have recognised as one of the dietary factors mainly responsible for the reduced mortality due to cardiovascular accidents in populations on a Mediterranean diet or on a diet rich in vegetable or plant substances and drinks such as wine and tea,

Studies carried out analysing the "French Paradox" phenomenon, i.e. the low mortality due to cardiovascular accidents in populations on a high-calorie diet rich in proteins and lipids, have identified olive oil, and, even more so, red wine, as the dietary factors capable of accounting for this apparent contradiction. Red wine, in fact, is rich in polyphenols endowed with a substantial antioxidant activity, particularly quercetin, myricectin, resveratrol and catechins.

Since the oxidation of LDLs plays an important role in the pathogenesis of atherosclerosis, the acknowledged antiatherogenic and vascular protective effects of red wine have been attributed to the presence of these polyphenols.

In particular, quercetin has been shown to inhibit not only LDL oxidation, but also LDL aggregation, which represents an additional modification of lipoproteins.

Recently, it has been found that extensive oxidation of LDLs leads to their aggregation and that both these modified forms of LDL are present in atherosclerotic lesions.

The antioxidant activity of quercetin and its protective activity against the production of free radicals have also been confirmed by tests conducted on Fe²⁺-dependent lipoperoxidation and on rat liver microsomes exposed to CCl₄.

Another important activity of quercetin, and one which may explain its cardio-protective activity, is its ability to affect in platelet aggregation. Quercetin, in fact, is capable of inhibiting platelet aggregation induced by thrombin or ADP as well as being capable of inhibiting platelet thromboxane synthesis and the synthesis of eicosanoids such as 12-HETE.

The inhibitory effect on platelet aggregation, and also on cyclic-AMP phosphodiasterase, may be due to its inhibition of the intracellular influx of calcium ions.

Tests on the vasorelaxant activity of quercetin, measured on the isolated aorta, have also demonstrated its ability to act as a vasodilator via enhanced nitric oxide synthesis.

To complete the pharmacological profile of quercetin, we should also recall its inhibitory activity on lipoperoxygenase and cyclo-oxygenase and their consequent allergic and inflammatory reactions, as well as its ability to potentiate prostacyclin with consequent cytoprotective and anti-inflammatory effects.

Another important characteristic of quercetin is that it is a selective inhibitor of tyrosine protein kinase and of activation of the nuclear transduction factor NFK-IKB, and thus, via this pathway, inhibits the formation of prostaglandins and cytokines as well as inhibiting tumour growth.

Numerous research studies, in fact, indicate that quercetin is capable of inhibiting the growth of leukaemic cells and the development of lung or breast tumours.

It has been demonstrated that quercetin is, in fact, a potent inhibitor of PI-kinase (1-phosphatidylinositol-4-kinase) and of PIP-kinase (phosphatidylinositol-4-phosphate-5-kinase) with consequent reduction of the second transduction messenger of the signal represented by IP3 (inositol-1,4,5-triphosphate), and its ability to inhibit tumour growth may be explained by this mechanism. Moreover, its ability to inhibit oestrone sulphatase may also be a further factor in explaining its ability to curb oestrogen-dependent tumour growth.

L-carnitine and its alkanoyl derivatives also play an important biological role in both the nutritional and therapeutic fields.

A deficiency of L-carnitine in the diet, as may occur in some cases in children, may slow down growth, which can be restored to normal by the administration of L-carnitine.

An L-carnitine deficiency in the body may lead to clinical syndromes of systemic type or syndromes confined to the myocardial or skeletal muscle systems. Among all the tissues of the body, the muscles and heart have the highest L-carnitine concentrations, which takes on very considerable physiological significance if we consider that the heart, above all, is strongly dependent for its energy requirements on the beta-oxidation of fatty acids, a process related to the presence of L-carnitine. In addition to its role as a carrier of long-chain fatty acids across the mitochondrial membrane, L-carnitine also plays an important role in blocking long-chain metabolites of acyl-CoA which may accumulate during states of tissue ischaemia and damage the sarcolemma in the muscles. It is also well known that an eccess of fatty acids during reperfusion may potentiate myocardial ischaemic damage.

L-carnitine and its alkanoyl derivates, in addition to playing a major role in the beta-oxidation of fats and in the energy production of ATP, are also capable of acting in energy production both in terms of glucose utilisation and in terms of the utilisation of branched-chain amino acids.

In the explanation of the complex pharmacological and therapeutic profile of the "carnitines", we must bear in mind not only their energy characteristics but also the data indicating their effective antioxidant action, as demonstrated by their protective effect against the lipoperoxidation of the cell phospholipid membranes as well as against the oxidative damage induced at myocardial or endothelial cell level.

It has now surprisingly been found that a composition containing a combination of the following as its characterising components:
(a) propionyl L-carnitine or a pharmacologically acceptable salt thereof, and
(b) a flavonoid selected from the group comprising quercetin, rutin, myricetin, myricitrin or mixtures thesereof,
is extremely effective in preventing and/or treating damage induced by the presence of free radicals and by increased platelet aggregation, as well as thrombotic or atherosclerotic abnormalities and allergic inflammatory reactions, as a result of the potent synergistic effect exerted by its components.

It has also been found that, advantageously, component (a) may further comprise of a "carnitine" selected from the group comprising L-carnitine, acetyl L-carnitine, valeryl L-carnitine and isovaleryl L-carnitine or a pharmacologically acceptable salt thereof, and that component (b) may consist of an extract of vegetable or plant products containing it.

The (a):(b) weight-to-weight ratio ranges fron 1:0.1 to 1:10.

The potent synergistic effect of the aforesaid components (a) and (b) has been ascertained by means of various pharmacological tests, some of which are reported here below.

### Toxicology

It is well known that both L-carnitine and its derivatives are characterised by low toxicity and excellent tolerability. Quercetin, too, like other naturally occurring polyphenols, presents very favourable toxicity and tolerability characteristics.

The tests performed combining the various "carnitines" with quercetin have confirmed the acknowledged good toxicity and tolerability characteristics of these compounds.

In tests performed in the rat, it proved possible to administer orally in a single adminstration up to 4 g/kg of propionyl L-carnitine or the same amount of a combination of acetyl L-carnitine, propionyl L-carnitine and isolvaleryl L-carnitine in a 1:1:1 weight-to-weight ratio without any signs of toxicity being observed. Similarly, no signs of toxicity were observed with the administration of a 1 g/kg dose of quercetin.

Similar favourable results were obtained with the administration of a combination of propionyl L-carnitine or carnitine mixture plus quercetin at the same doses indicated above. Even the prolonged administration of 1 g/kg of propionyl L-carnitine or the same amount of carnitine mixture in combination with 100 mg/kg of quercetin in rats for thirty days consecutively with the diet was well tolerated and led to no detectable toxic abnormalities in the animals thus treated.

The blood cell counts and tests for various biochemical parameters (serum glucose, BUN, cholesterol, triglycerides) revealed no abnormalities worthy of note as compared to control animals, and the histological examination carried out on the main organs (liver, kidneys, heart, lungs, brain) also failed to detect any pathological abnormalities, thus confirming the low toxicity and good tolerability of the new combination assessed in these tests as well.

### Platelet aggregation tests

Blood samples taken from healthy volunteers were used for these tests.

The blood samples were treated with sodium citrate and centrifuged for 8 minutes at 100 rpm. The number of platelets was counted and brought to a fixed level of 300,000 platelets/ml by adding platelet poor plasma (PPP) where necessary.

Platelet aggregation was induced using collagen (2,5 µg/ml, 5 µg/ml) as the aggregating agent and determined photometrically according to the method described by Born (Born G.V.R., Nature, 194, 927, 1962).

Platelet aggregation was measured in baseline conditions and after 10 minutes' incubation with quercetin or propionyl L-carnitine or with a combination of quercetin and propionyl L-carnitine in the same amounts.

The propionyl L-carnitine doses were 10 µg and 20 µg/ml, while the quercetin doses were 0.1 µg and 0.25 µg/ml. Whereas propionyl L-carnitine did not prove capable of modifying the platelet aggregating action induced by collagen, quercetin at the doses used (0.25 µg) reduced it by 50%, but the inhibition reached 100% when quercetin was combined with propionyl L-carnitine, thus demonstrating the potent synergistic effect which propioyl L-carnitine and quercetin are capable of exerting when used in combination.

### Antiatherosclerotic activity tests

In these tests, experimental atherosclerosis was induced in rabbits by administering 0.5% of cholesterol by weight together with the standard diet. New Zealand rabbits with a mean body weight of 2.8 kg were used in these tests and received, together with the cholesterol-enriched diet, 400 mg/kg of propionyl L-carnitine, or 400 mg/kg of carnitine mixture (propionyl L-carnitine, acetyl L-carnitine and isovaleryl L-carnitine in a 1:1:1 weight-to-weight ratio), or 50 mg/kg of quercetin, or various combinations of these products.

After thirty days' treatment, a blood sample was taken from the central artery of the ear in each animal and used to assay the lipoproteins present according to the method described by Hatch (Hatch F.T., Advan. Lipid Res., 6, 1, 1968).

The liver was then removed from each animal and used to assay total cholesterol and triglycerides according to the method of Dehoff (Dehoff J.L., Clin. Chem., 24, 433, 1978) and Levy (Levy A., Advances in Automated Analysis, 497 - Thurman - Miami, 1972). Arteriosclerotic lesions at the level of the heart and aorta were assessed according to the Klurfield method (Klurfield D.M., J. Med., 10, 35, 1979), grading them from I to IV according to the severity of the damage detected.

The results of these tests show that both propionyl L-carnitine and the carnitine mixture are capable of reducing both the biochemical and histological parameters of experimentally induced atherosclerosis in the rabbit.

Quercetin, too, shows good inhibitory activity. The greatest degree of protective activity, however, is that achieved when carnitine and quercetin are administered in combination. In this case, in fact, the atherosclerotic lesions either do not occur at all or are only minimally detectable.

These tests, too, demonstrate the potent synergistic effect that can be achieved by the combination of carnitines, and particularly propionyl L-carnitine, plus quercetin.

**Table 1**

| Plasma lipoprotein concentrations in hypercholesterolaemic rabbits | | | |
|---|---|---|---|
| | VLDL | LDL | HDL |
| | (mg/dl) | (mg/dl) | (mg/dl) |
| Hypercholesterolaemic controls | 1,220 ± 33.2 | 468 ± 22.8 | 25.2 ± 4.1 |
| Propionyl L-carnitine (400 mg/kg) | 855 ± 32.5 | 380 ± 21.5 | 26.4 ± 3.9 |
| Carnitine mixture (400 mg/kg) | 910 ± 41.8 | 345 ± 20.8 | 29.5 ± 4.4 |
| Quercetin (50 mg/kg) | 816 ± 37.5 | 298 ± 30.1 | 28.6 ± 3.8 |
| Propionyl L-carnitine (400 mg/kg) + Quercetin (50 mg/kg) | 318 ± 15.6 | 175 ± 20.2 | 30.8 ± 2.8 |
| Carnitine mixture (400 mg/kg) + Quercetin (50 mg/kg) | 378 ± 20.6 | 111 ± 10.2 | 31.1 ± 3.1 |

**Table 2**

| Liver concentrations of total cholesterol and triglycerides in hypercholesterolaemic rabbits | | |
|---|---|---|
| | Total cholesterol | Triglycerides |
| | (100 mg/g) | (mg/g) |
| Hypercholesterolaemic controls | 1,915 ± 220 | 190 ± 16.3 |
| Propionyl L-carnitine (400 mg/kg) | 1,470 ± 310 | 165 ± 14.5 |
| Carnitine mixture (400 mg/kg) | 1,495 ± 355 | 145 ± 15.9 |
| Quercetin (50 mg/kg) | 1,320 ± 230 | 130 ± 14.5 |
| Propionyl L-carnitine (400 mg/kg) + Quercetin (50 mg/kg) | 720 ± 65 | 105 ± 9.62 |
| Carnitine mixture (400 mg/kg) + Quercetin (50 mg/kg) | 795 ± 72 | 115 ± 11.2 |

### Anti-inflammatory activity tests

To assess the anti-inflammatory activity of the combination of propionyl L-carnitine or carnitine mixture plus quercetin, its inhibitory effect on oedema induced in the rat paw by subplantar injection of carrageenin was evaluated.

To this end, 0.1 cc of a 1% carrageenin solution (Sigma, St, Louis, USA) were injected in the subplantar zone of the rat paw. The volume of oedema of the paw was measured by means of a mercury plethysmograph at intervals of one hour over the four-hour period following injection of carrageenin. One hour prior to injection of carrageenin the animals received oral administrations either of propionyl L-carnitine (300 mg/kg and 150 mg/kg), or carnitine mixture (acetyl L-carnitine, propionyl L-carnitine, isovaleryl L-carnitine; 300 mg/kg and 150 mg/kg), or quercetin (100 mg/kg and 50 mg/kg), or various combinations of these compounds. The results of these tests indicate that whereas propionyl L-carnitine has a modest anti-oedema effect, as does quercetin, when propionyl L-carnitine or the carnitine mixture is combined with quercetin the anti-inflammatory effect becomes very marked, thus demonstrating, in this case, too, that a potent synergistic effect of quercetin and carnitines is achieved.

**Table 3**

| Anti-inflammatory activity tests | | | | | |
|---|---|---|---|---|---|
| Treatment | mg/kg | % reduction of carrageenin-induced oedema after | | | |
| | | 1 | 2 | 3 | 4h |
| Propionyl L-carnitine | 150 | --- | --- | --- | --- |
| Propionyl L-carnitine | 300 | 12 ± 0.1 | 10 ± 0.2 | 6 ± 0.5 | --- |
| Carnitine mixture | 150 | --- | --- | --- | --- |
| Carnitine mixture | 300 | --- | 12 ± 0.3 | 5 ± 0.7 | --- |
| Quercetin | 50 | 8 ± 0.6 | 10 ± 0.2 | --- | --- |
| Quercetin | 100 | 18 ± 1.2 | 22 ± 2.5 | 20 ± 1.9 | 18 ± 1.5 |
| Propionyl L-carnitine + Quercetin | 150 + 50 | 22 ± 2.4 | 26 ± 3.1 | 25 ± 2.5 | 20 ± 3.9 |
| Carnitine mixture + Quercetin | 150 + 50 | 20 ± 3.1 | 25 ± 2.9 | 20 ± 2.5 | 18 ± 3.1 |
| Propionyl L-carnitine + Quercetin | 300 + 100 | 36 ± 4.2 | 39 ± 4.2 | 34 ± 3.8 | 30 ± 2.9 |
| Carnitine mixture + Quercetin | 300 + 100 | 37 ± 3.9 | 35 ± 4.7 | 35 ± 4.1 | 32 ± 3.9 |

### Anaphylactic shock tests

These tests were performed using male albino guinea-pigs with a mean weight of 300 g; the animals received intraperitoneal injections of 1 cc of horse serum diluted 1:10.

After twenty-five days, intravenous injection of 1 cc of horse serum triggered anaphylactic shock with onset of bronchospasm and death of the animals. Five days before the shock-triggering injection, the sensitised animals were treated orally either with propionyl L-carnitine (300 mg/kg), or with carnitine mixture (300 mg/kg), or with quercetin (50 mg/kg), or with various combinations of these compounds.

It was found that the administration of the combination of propionyl L-carnitine and quercetin and the combination of carnitine mixture and quercetin is capable of protecting more than half the treated animals against death by anaphylactic shock, whereas no protective effect is observed with administration of either propionyl L-carnitine or quercetin alone, thus demonstrating that in these tests, too, there was a potent synergistic effect between carnitines and quercetin.

**Table 4**

| Protection against anaphylactic shock. | |
|---|---|
| Treatment | Surviving/treated animals |
| Propionyl L-carnitine (300 mg/kg) | 2/10 |
| Carnitine mixture (300 mg/kg) | 1/10 |
| Quercetin (50 mg/kg) | 3/10 |
| Propionyl L-carnitine (300 mg/kg) + Quercetin (50 mg/kg) | 6/10 |
| Carnitine mixture (300 mg/kg) + Quercetin (50 mg/kg) | 5/10 |

### Tests on leukopenia induced by mitomycin C

To evaluate the immunostimulatory effect of the combination of propionyl L-carnitine or carnitine mixture plus quercetin, the effect of these products was evaluated on the toxic and immunosuppressive activity induced by mitomycin C.

Mitomycin C (50 µg/mouse) injected intraperitoneally in mice every day for five consecutive days causes severe leukopenia which then worsens progressively up to the death of the animals which occurs after approximately 12 days.

The oral administration of propionyl L-carnitine (300 mg/kg), or of carnitine mixture (300 mg/kg), or of quercetin (100 mg/kg), or of these substances in combination, from the first day of administration of mitomycin C up to day five, inhibits the reduction of leukocytes and increases the survival time of the animals thus treated.

The protective effect is modest with administration of propionyl L-carnitine, carnitine mixture or quercetin alone, but becomes very marked with the combinations of propionyl L-carnitine and carnitine mixture plus quercetin. The results of these tests again demonstrate a protective synergistic effect of the combination of propionyl L-carnitine plus quercetin, in this case against the immunosuppressive and toxic action of mitomycin C.

**Table 5**

| Effect on leukopenia and survival time in mice treated with mitomycin C. | | | | | | |
|---|---|---|---|---|---|---|
| Treatment | Number of leukocytes after (days) | | | % Rats surviving after (days) | | |
| | 5 | 10 | 12 | 5 | 10 | 12 |
| Mitomycin C | 5,800 ± 250 | 3,200 ± 310 | 1.200 ± 220 | 60 | 35 | 20 |
| Propionyl L-carnitine | 6,000 ± 310 | 5,400 ± 280 | 2,100 ± 125 | 60 | 45 | 30 |
| Carnitine mixture | 6,200 ± 405 | 4,300 ± 340 | 2,200 ± 270 | 70 | 35 | 30 |
| Quercetin | 5,600 ± 480 | 4,800 ± 410 | 2,800 ± 310 | 70 | 30 | 30 |
| Propionyl L-carnitine + Quercetin | 7,100 ± 410 | 6,200 ± 370 | 6,100 ± 380 | 90 | 75 | 65 |
| Carnitine mixture + Quercetin | 6,800 ± 510 | 6,000 ± 420 | 5,800 ± 480 | 80 | 80 | 60 |

Illustrative, non-limiting examples of compositions according to the invention are reported hereinbelow.

| | | | |
|---|---|---|---|
| 1) | Propionyl L-carnitine | mg | 500 |
| | Quercetin | mg | 250 |
| | | | |
| 2) | Carnitine mixture (propionyl L-carnitine mg 125, acetyl L-carnitine mg 125, isovaleryl L-carnitine mg 125) | mg | 375 |
| | Quercetin | mg | 125 |
| | | | |
| 3) | Propionyl L-carnitine | mg | 250 |
| | Quercetin | mg | 125 |
| | | | |
| 4) | Carnitine mixture (propionyl L-carnitine mg 75, acetyl L-carnitine mg 75, isovaleryl L-carnitine mg 75) | mg | 225 |
| | Quercetin | mg | 125 |
| 5) | Propionyl L-carnitine | mg | 125 |
| | Quercetin | mg | 125 |
| | Citroflavonoids | mg | 50 |
| | Vit. C | mg | 100 |
| | Rutin | mg | 20 |
| | CoQ₁₀ | mg | 10 |
| | Vit. E | mg | 5 |
| | β-carotene | mg | 6 |
| | Manganese glycinate | mg | 5 |
| | Zinc glycinate | mg | 5 |
| | Magnesium glycinate | mg | 20 |
| | Selenium methionine | µg | 50 |
| | | | |
| (6) | Carnitine mixture | mg | 300 |
| | (propionyl L-carnitine mg 100, acetyl L-carnitine mg 100, isovaleryl L-carnitine mg 100) | | |
| | Quercetin | mg | 150 |
| | Citroflavonoids | mg | 50 |
| | Vit. C | mg | 100 |
| | Rutin | mg | 20 |
| | CoQ₁₀ | mg | 10 |
| | Vit. E | mg | 5 |
| | β-carotene | mg | 5 |
| | Manganese glycinate | mg | 5 |
| | Zinc glycinate | mg | 5 |
| | Magnesium glycinate | mg | 20 |
| | Selenium methionine | µg | 50 |

What is meant by pharmacologically acceptable salt of L-carnitine or alkanoyl L-carnitine is any salt of these active ingredients with an acid that does not give rise to unwanted toxic or side effects. These acids are well known to pharmacy experts.

Non-limiting examples of suitable salts are the following: chloride; bromide; iodide; aspartate, acid aspartate; citrate, acid citrate; tartrate; phosphate, acid phosphate; fumarate; acid fumarate; glycerophosphate; glucose phosphate; lactate; maleate, acid maleate; orotate; oxalate, acid oxalate; sulphate, acid sulphate, trichloroacetate, trifluoroacetate and methanesulphonate.

A list of FDA-approved pharmacologically acceptable salts is given in Int. J. of Pharm. 33, (1986), 201-217.

## Claims

1. Use of:
(a) propionyl L-carnitine or a pharmacologically acceptable salt thereof; and
(b) a flavonoid in a synergistically effective weight ratio, for producing a combination composition, a medicament or a dietary supplement for the prevention and treatment of diseases brought about by the presence of free radicals and by increased platelet aggregation, thrombotic or atherosclerotic abnormalities, allergic inflammatory reactions.

2. The use according to claim 1, wherein the ingredient (a) further comprises a "carnitine" selected from the group comprising L-carnitine, acetyl L-carnitine, valeryl L-carnitine, isovaleryl L-carnitine or their pharmacologically acceptable salts or mixtures thereof.

3. The use according to claim 1 or 2, wherein the flavonoid is selected from the group comprising quercetin, rutin, myricetin and myricitrin.

4. The use according to any of claims 1-3, wherein the weight ratio (a): (b) is from 1: 0.1 to 1: 10.

5. The use according to any of the preceding claims, wherein the ingredient (b) is in the form of vegetal extracts which contain the ingredient itself.

6. The use according to any of the preceding claims, wherein the pharmacologically acceptable salt of L-carnitine or alkanoyl L-carnitine is selected from the group comprising: chloride; bromide; iodide; aspartate, acid aspartate; citrate, acid citrate; tartrate; phosphate, acid phosphate; fumarate, acid fumarate; glycerophosphate; glucose phosphate; lactate; maleate, acid maleate; orotate; acid oxalate; sulphate, acid sulphate; trichloroacetate; trifluoroacetate and methane sulphonate.

7. The use according to any of the preceding claims, wherein the medicament or the dietary supplement further comprises vitamins, coenzymes, mineral substances and antioxidants.

8. The use according to any of the preceding claims, wherein the dietary supplement is in a orally administrable form.

9. The use according to any of the preceding claims, wherein the medicament is in an orally, parenterally, rectally, cutaneously, ocularly or transdermally administrable form.

10. The use according to claim 8, wherein the dietary supplement is in solid, semi-solid or liquid form.

11. The use according to claim 9, wherein the medicament is in solid, semi-solid or liquid form.

12. The use according to claim 10, wherein the dietary supplement is in the form of tablets, lozenges, pills, capsules, granulates, syrups, vials or drops.

13. The use according to claim 11, wherein the medicament is in the form of tablets, lozenges, pills, capsules, granulates, syrups, vials or drops.

14. The use according to any of the preceding claims, wherein the composition, dietary supplement or the medicament is in a unit dosage form, comprising:
| | |
|---|---|
| Propionyl L-carnitine | mg 500 |
| Quercetin | mg 250. |

15. The use according to any of claims 1-14, wherein the composition, dietary supplement or the medicament is in unit dosage form, comprising:
| | |
|---|---|
| Carnitine mixture (propionyl L-carnitine mg 125, acetyl L-carnitine mg 125, isovaleryl L-carnitine mg 125) | mg 375 |
| Quercetin | mg 250. |

16. The use according to any of claims 1-14, wherein the composition, dietary supplement or the medicament is in unit dosage form, comprising:
| | |
|---|---|
| Propionyl L-carnitine | mg 250 |
| Quercetin | mg 125. |

17. The use according to any of claims 1-14, wherein the composition, dietary supplement or the medicament is in unit dosage form, comprising:
| | |
|---|---|
| Carnitine mixture (propionyl L-carnitine mg 75, acetyl L-carnitine mg 75, isovaleryl L-carnitine mg 75) | mg 225 |
| Quercetin | mg 125. |

18. The use according to claim 1, wherein the composition, dietary supplement or the medicament is in unit dosage form, comprising:
| | |
|---|---|
| Propionyl L-carnitine | mg 125 |
| Quercetin | mg 125 |
| Citroflavonoids | mg 50 |
| Vit. C | mg 100 |
| Rutin | mg 20 |
| CoQ₁₀ | mg 10 |
| Vit. E | mg 5 |
| β-carotene | mg 5 |
| Manganese glycinate | mg 5 |
| Zinc glycinate | mg 5 |
| Magnesium glycinate | mg 20 |
| Selenium methionine | µg 50. |

19. The use according to any of claims 1-14, wherein the composition, dietary supplement or the medicament is in unit dosage form, comprising:
| | |
|---|---|
| Carnitine mixture (propionyl L-carnitine mg 100, (propionyl L-carnitine mg 100, (propionyl L-carnitine mg 100, acetyl L-carnitine mg 100, isovaleryl L-carnitine mg 100) | mg 300 |
| Quercetin | mg 150 |
| Citroflavonoids | mg 50 |
| Vit. C | mg 100 |
| Rutin | mg 20 |
| CoQ₁₀ | mg 10 |
| Vit. E | mg 5 |
| β-carotene | mg 5 |
| Manganese glycinate | mg 5 |
| Zinc glycinate | mg 5 |
| Magnesium glycinate | mg 20 |
| Selenium methionine | µg 50. |

## Patentansprüche

1. Verwendung von
(a) Propionyl-L-carnitin oder eines pharmakologisch akzeptablen Salzes davon und
(b) eines Flavonoids in einem synergistisch wirksamen Gewichtsverhältnis zur Herstellung einer Kombinationszusammensetzung, eines Medikaments oder eines Nahrungsergänzungsmittels für die Prävention und Behandlung von Krankheiten, die durch das Vorliegen von freien Radikalen und durch erhöhte Thrombozytenaggregation, thrombotische oder atherosklerotische Abnormalitäten, allergische Entzündungsreaktionen verursacht werden.

2. Verwendung nach Anspruch 1, wobei das Ingrediens (a) außerdem ein "Carnitin", ausgewählt aus der Gruppe umfassend L-Carnitin, Acetyl-L-carnitin, Valeryl-L-carnitin, Isovaleryl-L-carnitin oder deren pharmakologisch akzeptablen Salze oder Gemische davon, umfasst.

3. Verwendung nach Anspruch 1 oder 2, wobei das Flavonoid aus der Gruppe, umfassend Quercetin, Rutin, Myricetin und Myricitrin, ausgewählt ist.

4. Verwendung nach einem der Ansprüche 1 bis 3, wobei das Gewichtsverhältnis (a) : (b) 1 : 0,1 bis 1 : 10 ist.

5. Verwendung nach einem der vorangehenden Ansprüche, wobei das Ingrediens (b) in Form von vegetabilischen Extrakten ist, die das Ingrediens selbst enthalten.

6. Verwendung nach einem der vorangehenden Ansprüche, wobei das pharmakologisch akzeptable Salz von L-Carnitin oder Alkanoyl-L-carnitin aus der Gruppe ausgewählt ist, die umfasst: Chlorid; Bromid; Iodid; Aspartat, saures Aspartat; Citrat, saures Citrat; Tartrat; Phosphat, saures Phosphat; Fumarat, saures Fumarat; Glycerophosphat; Glucosephosphat; Lactat; Maleat, saures Maleat; Orotat; saures Oxalat; Sulfat, saures Sulfat; Trichloracetat; Trifluoracetat und Methansulfonat.

7. Verwendung nach einem der vorangehenden Ansprüche, wobei das Medikament oder das Nahrungsergänzungsmittel außerdem Vitamine, Coenzyme, Mineralsubstanzen und Antioxidanzien umfasst.

8. Verwendung nach einem der vorangehenden Ansprüche, wobei das Nahrungsergänzungsmittel in einer oral verabreichbaren Form ist.

9. Verwendung nach einem der vorangehenden Ansprüche, wobei das Medikament in einer oral, parenteral, rektal, kutan, okular oder transdermal verabreichbaren Form ist.

10. Verwendung nach Anspruch 8, wobei das Nahrungsergänzungsmittel in fester, halbfester oder flüssiger Form ist.

11. Verwendung nach Anspruch 9, wobei das Medikament in fester, halbfester oder flüssiger Form ist.

12. Verwendung nach Anspruch 10, wobei das Nahrungsergänzungsmittel in Form von Tabletten, Lutschbonbons, Pillen, Kapseln, Granulaten, Sirupen, Phiolen oder Tropfen vorliegt.

13. Verwendung nach Anspruch 11, wobei das Medikament in Form von Tabletten, Lutschbonbons, Pillen, Kapseln, Granulaten, Sirupen, Phiolen oder Tropfen vorliegt.

14. Verwendung nach einem der vorangehenden Ansprüche, wobei die Zusammensetzung, das Nahrungsergänzungsmittel oder das Medikament in Einheitsdosierungsform ist, umfassend:
| | |
|---|---|
| Propionyl-L-carnitin | 500 mg |
| Quercetin | 250 mg. |

15. Verwendung nach einem der Ansprüche 1 bis 14, wobei die Zusammensetzung, das Nahrungsergänzungsmittel oder das Medikament in Einheitsdosierungsform ist, umfassend:
| | | |
|---|---|---|
| Carnitingemisch | | 375 mg |
| (Propionyl-L-carnitin | 125 mg, | |
| Acetyl-L-carnitin | 125 mg, | |
| Isovaleryl-L-carnitin | 125 mg) | |
| Quercetin | | 250 mg. |

16. Verwendung nach einem der Ansprüche 1 bis 14, wobei die Zusammensetzung, das Nahrungsergänzungsmittel oder das Medikament in Einheitsdosierungsform ist, umfassend:
| | |
|---|---|
| Propionyl-L-carnitin | 250 mg |
| Quercetin | 125 mg. |

17. Verwendung nach einem der Ansprüche 1 bis 14, wobei die Zusammensetzung, das Nahrungsergänzungsmittel oder das Medikament in Einheitsdosierungsform ist, umfassend:
| | | |
|---|---|---|
| Carnitingemisch | | 225 mg |
| (Propionyl-L-carnitin | 75 mg, | |
| Acetyl-L-carnitin | 75 mg, | |
| Isovaleryl-L-carnitin | 75 mg) | |
| Quercetin | | 125 mg. |

18. Verwendung nach Anspruch 1, wobei die Zusammensetzung, das Nahrungsergänzungsmittel oder das Medikament in Einheitsdosierungsform ist, umfassend:
| | |
|---|---|
| Propionyl-L-carnitin | 125 mg |
| Quercetin | 125 mg |
| Citroflavonoide | 50 mg |
| Vitamin C | 100 mg |
| Rutin | 20 mg |
| CoQ₁₀ | 10 mg |
| Vitamin E | 5 mg |
| β-Carotin | 5 mg |
| Manganglycinat | 5 mg |
| Zinkglycinat | 5 mg |
| Magnesiumglycinat | 20 mg |
| Selenmethionin | 50 µg |

19. Verwendung nach einem der Ansprüche 1 bis 14, wobei die Zusammensetzung, das Nahrungsergänzungsmittel oder das Medikament in Einheitsdosierungsform ist, umfassend:
| | | |
|---|---|---|
| Carnitingemisch | | 300 mg |
| (Propionyl-L-carnitin | 100 mg, | |
| Acetyl-L-carnitin | 100 mg, | |
| Isovaleryl-L-carnitin | 100 mg) | |
| Quercetin | | 150 mg |
| Citroflavonoide | | 50 mg |
| Vitamin C | | 100 mg |
| Rutin | | 20 mg |
| CoQ₁₀ | | 10 mg |
| Vitamin E | | 5 mg |
| β-Carotin | | 5 mg |
| Manganglycinat | | 5 mg |
| Zinkglycinat | | 5 mg |
| Magnesiumglycinat | | 20 mg |
| Selenmethionin | | 50 µg |

## Revendications

1. Utilisation :
(a) de la propionyl-L-carnitine ou d'un sel pharmacologiquement acceptable de celle-ci ; et
(b) d'un flavonoïde dans un rapport massique efficace du point de vue de la synergie,
pour la préparation d'une composition de combinaison, d'un médicament ou d'un complément alimentaire, destiné à la prévention et au traitement de maladies provoquées par la présence de radicaux libres et par une agrégation plaquettaire accrue, d'anomalies thrombotiques ou athérosclérotiques, de réactions inflammatoires allergiques.

2. Utilisation selon la revendication 1, dans laquelle le composé (a) comprend en outre une "carnitine" choisie parmi le groupe comprenant la L-carnitine, l'acétyl-L-carnitine, la valéryl-L-carnitine, l'isovaléryl-L-carnitine ou leurs sels pharmacologiquement acceptables ou des mélanges de ceux-ci.

3. Utilisation selon la revendication 1 ou 2, dans laquelle le flavonoïde est choisi dans le groupe comprenant la quercétine, la rutine, la myricétine et la myricitrine.

4. Utilisation selon l'une quelconque des revendications 1 à 3, dans laquelle le rapport massique (a):(b) varie de 1:0,1 à 1:10.

5. Utilisation selon l'une quelconque des revendications précédentes, dans laquelle le composé (b) est sous la forme d'extraits végétaux contenant le composé lui-même.

6. Utilisation selon l'une quelconque des revendications précédentes, dans laquelle le sel pharmacologiquement acceptable de la L-carnitine ou de la L-carnitine alcanoylée est choisi parmi le groupe comprenant : le chlorure ; le bromure ; l'iodure ; l'aspartate, l'aspartate acide ; le citrate, le citrate acide ; le tartrate ; le phosphate, le phosphate acide ; le fumarate, le fumarate acide ; le glycérophosphate ; le phosphate de glucose ; le lactate ; le maléate, le maléate acide ; l'orotate ; l'oxalate acide ; le sulfate, le sulfate acide ; le trichloroacétate ; le trifluoroacétate et le sulfonate de méthane.

7. Utilisation selon l'une quelconque des revendications précédentes, dans laquelle le médicament ou le complément alimentaire comprend en outre des vitamines, des co-enzymes, des substances minérales et des antioxydants.

8. Utilisation selon l'une quelconque des revendications précédentes, dans laquelle le complément alimentaire est sous une forme administrable par voie orale.

9. Utilisation selon l'une quelconque des revendications précédentes, dans laquelle le médicament est sous une forme administrable par voie orale, parentérale, rectale, cutanée, oculaire ou transdermique.

10. Utilisation selon la revendication 8, dans laquelle le complément alimentaire est sous forme solide, semi-solide ou liquide.

11. Utilisation selon la revendication 9, dans laquelle le médicament est sous forme solide, semi-solide ou liquide.

12. Utilisation selon la revendication 10, dans laquelle le complément alimentaire est sous la forme de comprimés, de tablettes, de pilules, de gélules, de granules, de sirops, d'ampoules ou de gouttes.

13. Utilisation selon la revendication 11, dans laquelle le médicament est sous la forme de comprimés, de tablettes, de pilules, de gélules, de granules, de sirops, d'ampoules ou de gouttes.

14. Utilisation selon l'une quelconque des revendications précédentes, dans laquelle la composition, le complément alimentaire ou le médicament est sous forme de dose unitaire, comprenant:
| | |
|---|---|
| Propionyl-L-carnitine | 500 mg |
| Quercétine | 250 mg |

15. Utilisation selon l'une quelconque des revendications 1 à 14, dans laquelle la composition, le complément alimentaire ou le médicament est sous forme de dose unitaire, comprenant :
| | | |
|---|---|---|
| Mélange de carnitines | | 375 mg |
| (propiocyl-L-carnitine | 125 mg, | |
| acétyl-L-carnitine | 125 mg, | |
| isovaléryl-L-carnitine | 125 mg) | |
| Quercétine | | 250 mg |

16. Utilisation selon l'une quelconque des revendications 1 à 14, dans laquelle la composition, le complément alimentaire ou le médicament est sous forme de dose unitaire, comprenant :
| | |
|---|---|
| Propionyl-L-carnitine | 250 mg |
| Quercétine | 125 mg |

17. Utilisation selon l'une quelconque des revendications 1 à 14, dans laquelle la composition, le complément alimentaire ou le médicament est sous forme de dose unitaire, comprenant :
| | | |
|---|---|---|
| Mélange de carnitines | | 225 mg |
| (propionyl-L-carnitine | 75 mg, | |
| acétyl-L-carnitine | 75 mg, | |
| isovaléryl-L-carnitine | 75 mg) | |
| Quercétine | | 125 mg |

18. Utilisation selon la revendication 1, dans laquelle la composition, le complément alimentaire ou le médicament est sous forme de dose unitaire, comprenant :
| | |
|---|---|
| Propionyl-L-carnitine | 125 mg |
| Quercétine | 125 mg |
| Citroflavonoïdes | 50 mg |
| Vitamine C | 100 mg |
| Rutine | 20 mg |
| CoQ₁₀ | 10 mg |
| Vitamine E | 5 mg |
| β-carotène | 5 mg |
| Glycinate de manganèse | 5 mg |
| Glycinate de zinc | 5 mg |
| Glycinate de magnésium | 20 mg |
| Méthionine de sélénium | 50 µg |

19. Utilisation selon l'une quelconque des revendications 1 à 14, dans laquelle la composition, le complément alimentaire ou le médicament est sous forme de dose unitaire, comprenant :
| | | |
|---|---|---|
| Mélange de carnitines | | 300 mg |
| (propionyl-L-carnitine | 100 mg, | |
| acétyl-L-carnitine | 100 mg, | |
| isovaléryl-L-carnitine | 100 mg) | |
| Quercétine | | 150 mg |
| Citroflavonoïdes | | 50 mg |
| Vitamine C | | 100 mg |
| Rutine | | 20 mg |
| CoQ₁₀ | | 10 mg |
| Vitamine E | | 5 mg |
| β-carotène | | 5 mg |
| Glycinate de manganèse | | 5 mg |
| Glycinate de zinc | | 5 mg |
| Glycinate de magnésium | | 20 mg |
| Méthionine de sélénium | | 50 µg |
